Europäisches Patentamt

European Patent Office

Office européen des brevets

(11) Publication number: **0 288 104 B1**

## EUROPEAN PATENT SPECIFICATION

(45) Date of publication of patent specification: **10.06.92**  (51) Int. Cl.⁵: **C07C 321/18**, C07C 319/00

(21) Application number: **88200629.9**

(22) Date of filing: **31.03.88**

(54) **Process for the preparation of disulphides.**

(30) Priority: **15.04.87 GB 8709060**

(43) Date of publication of application:
**26.10.88 Bulletin 88/43**

(45) Publication of the grant of the patent:
**10.06.92 Bulletin 92/24**

(84) Designated Contracting States:
**AT BE CH DE ES FR GB GR IT LI LU NL SE**

(56) References cited:
**GB-A- 1 122 889**
**GB-A- 1 260 030**
**US-A- 3 565 959**
**US-A- 4 288 627**

(73) Proprietor: **SHELL INTERNATIONALE RE-
SEARCH MAATSCHAPPIJ B.V.**
**Carel van Bylandtlaan 30**
**NL-2596 HR Den Haag(NL)**

(72) Inventor: **Van Kruchten, Eugenè Marie God-
fried André**
**Badhuisweg 3**
**NL-1031 CM Amsterdam(NL)**
Inventor: **Leenaars, Corrie Maria Jeanne**
**Badhuisweg 3**
**NL-1031 CM Amsterdam(NL)**

## Description

The present invention relates to the preparation of a disulphide by oxidation of a thiol.

The oxidation of thiols has been described in Rec. trav. chim. Pays Bas, 82 (1963) pp. 773-789, which article discloses the formation of disulphides, together with sulphinic and sulphonic acids when the oxidation is carried out in tert-butanol. The oxidation of thiols and disulphides to sulphonic acids in dimethylformamide (DMF) has been described in Tetrahedron Lett., 1963, pp. 1131-1136. The oxidation of thiols in alkaline aqueous solutions has been disclosed in J. Appl. Chem, 18 (1968) pp. 330-335 and 335-339. The reaction was run in the absence of added metal catalysts (pp. 330-335) and in the presence thereof (pp. 335-339). The reaction rate was enhanced considerably by the addition of various metal catalyst. The products obtained consisted almost exclusively of disulphides.

In general, the prior art teaches the oxidation of thiols leads almost exclusively to the production of disulphides when the oxidation occurs in aqueous solutions, and to a variety of disulphides, sulphenic, sulphinic and sulphonic acids, when carried out in non aqueous solutions (see Adv. Chem. Ser., 75 (1968) p. 216). However, the use of an aqueous system has the disadvantages that the water-insoluble products tend to cause difficulties during the reaction by hindering the stirring of the mixture and the dispersion of an oxidizing gas (oxygen, air) when use is made thereof. Further, after separation of the product, e.g. by filtration, careful washing is needed to remove the base which produced the alkalinity of the reaction mixture. Moreover, it is evident that in an industrial process preference is given to the handling of a liquid product rather than a solid product. The present invention provides a process which selectively produces disulphides and which minimises the above drawbacks.

GB-A-1,122,889 states that, in general, liquefied petroleum gas, straight run gasoline, naphtha, kerosene, gas oil and other petroleum fractions obtained by distillation of a crude oil, cracked gasoline, cracked gas oil and other fractions obtained by thermal or catalytic cracking, and the products of other cracking processes such as coking of a crude oil, always contain mercaptans and that these mercaptans have harmful properties such as corrosiveness, and are malodorous. In order to eliminate the harmful and malodorous properties of mercaptans, there is provided a process for oxidising mercaptans which comprises subjecting a mercaptan to oxidation by means of oxygen in the presence of a catalyst composition comprising a sulphur dye and a metal or metal compound.

US-A-3,565,959 discloses a process for oxidising mercaptans having harmful properties and malodours to facilitate removal thereof by converting them into disulphides by oxidation thereof which comprises bringing mercaptans into contact with an oxidising agent such as oxygen or oxygen-containing gases in the presence of a poly metalo-phthalocyanine compound obtained by reacting at least a metal salt of metals of iron, copper, cobalt, nickel, chromium, manganese, zinc, vanadium and molybdenum with pyromellitonitrile, or, a mixture of urea and any one of pyromellitic anhydride, pyromellitic acid or pyromellitic acid imide.

Accordingly, the present invention provides a process for the preparation of disulphides which comprises oxidizing a thiol in an alkaline aqueous solution in the presence of a substantially water-immiscible organic solvent, a metal catalyst, and a phase transfer catalyst, the third concentration ranging from 20 to 40%m, based on organic solvent; after completion of reaction, separating aqueous and organic liquid phases, and isolating disulphide by removal of organic solvent from the separated organic liquid phase. This process gives excellent yields of disulphides with little or no solid material, due to the presence of the organic solvent, so that gas dispersion, if any, and stirring of the mixture is facilitated. The process is carried out in the presence of a phase transfer catalyst, since this significantly increases the reaction rate.

The reaction is carried out in the presence of a metal catalyst, since this accelerates the reaction rate. A wide variety of metals can be used, suitable metals including those of the following groups of the Periodic Table of the Elements: Groups 5, 6, 7, 8, 9, 10, 11, 12, 13 and 14 and the Lanthanides and Actinides. (This notation of the groups is taken from e.g. Chemical and Engineering News, 63(5) (1985) p. 27 and corresponds with the previous IUPAC notation of the groups VA(5), VIA(6), VIIA(7), VIIIA(8, 9, 10), IB (11), IIB(12), IIIB(13) and IVB(14)). Preferably the metal catalyst is present as a cation, and comprises at least one metal selected from cerium, uranium, vanadium, chromium, molybdenum, tungsten, manganese, iron, cobalt, nickel, palladium, platinum, copper, silver, zinc, cadmium, mercury, aluminium, thallium and tin. The most preferred metal catalysts are cobalt, nickel and/or copper; in particular copper.

The oxidizing agent used in the present process can be selected from a wide range of oxidants, such as permanganate, peroxides, iodine and the like, with iodine or gaseous oxygen being preferred. The oxygen can be used in pure form or in dilution with inert gases, e.g. such as in air, which is generally preferred for economic reasons.

The reaction is carried out in an alkaline aqueous solution. The alkalinity can be provided by a variety of bases, such as metal oxides, hydroxides or carbonates, ammonia and organic amines. The metals used in the bases can suitably be selected from the alkali and alkaline earth metals. Since alkali and alkaline earth metal hydroxides and ammonia are relatively strong bases and relatively cheap compounds, preference is given to the use of these compounds to render the reaction mixture alkaline. The ratio of the base and the thiol is not critical provided that the solution is alkaline. Preferably the equivalent ratio of the base, such as the alkali or alkaline earth metal hydroxide or ammonia, to the thiol is at least equal to 1. For economic reasons the ratio conveniently does not exceed 10. Hence, the equivalent ratio is advantageously from 1.5 to 10, in particular from 2 to 4.5.

The substantially water-immiscible organic solvent is used to provide a suitable solvent for the thiol and in particular for the disulphides formed. The expert in the field can easily establish which solvent would be best, in view of the thiol to be oxidized. Suitable organic solvents include (cyclo)aliphatic hydrocarbons, halogenated (cyclo)aliphatic hydrocarbons, aromatic hydrocarbons, halogenated aromatic hydrocarbons, alkylated aromatic hydrocarbons, ethers, petroleum ethers and mixtures thereof. Examples of such suitable solvents are pentane, hexane, cyclohexane, heptane, carbon tetrachloride, benzene, toluene, xylene, ethylbenzene, diethylether, methylbutylether mineral lubricating oils and the various commercial petroleum ethers. Preference is given to the use of benzene, toluene and/or xylene. The volume ratio of the aqueous solution to the substantially water-immiscible organic solvent is not critical. Factors which can influence this ratio include the base concentration in the aqueous phase, the solubility of the thiol and the disulphide to be formed in the organic solvent and water, and whether the disulphide to be formed is solid or liquid under the reaction contions. The expert in the field is able to select a suitable ratio of aqueous solution to organic solvent. Preferably this volume ratio ranges form 4:1 to 1:1.

Phase transfer catalyst is used to denote any reagent which will accelerate interphase reactions in aqueous/organic two-phase systems. Suitable such reagents include quaternary ammonium compounds, quaternary phosphonium compounds, crown ethers and cryptates. An examples of a suitable cryptate is the compound called "Kryptofix 222", being 1,10-diaza-4,7,13,16,21,24-hexaoxabicyclo[8.8.8]-hexaeicosane. Crown ethers are macrocyclic polyethers and are commonly designated by reference to the total number of atoms forming the macrocyclic ring together with the number of oxygen atoms in that ring. Thus, the macrocyclic polyether 1,4,7,10,13, 16-hexaoxacyclo octadecane is designated as 18-crown-6. This 18-crown-6 and 15-crown-5 and derivatives thereof are suitable examples of crown ethers. Suitable phosphonium compounds are, for example, tetramethylphosphonium and tetra-n-butylphosphonium chloride and bromide. Preferably, the phase transfer catalyst is a quaternary ammonium compound. Suitable examples of such ammonium compounds include tetra($C_{1-12}$)-alkyl ammonium halides and hydroxides in which one or more alkyl groups can be substituted by one or more phenyl groups. These preferred compounds include methyl tri($C_{8-10}$)alkylammonium halide or hydroxide, benzyltri($C_{1-2}$)ammonium halide or hydroxide, tetra($C_{1-4}$)ammonium halide or hydroxide and cetyltrimethylammonium halide or hydroxide, though aryl group-containing ammonium compounds such as methyltri(2-methylphenyl)ammonium halide or hydroxide can also be employed. As halides preferably the chlorides or bromides are used. Other types of compound which may be used as the phase transfer catalyst include quaternary ammonium anion exchange resins (suitably in the hydroxyl form).

The concentration of the phase transfer catalyst may vary widely, but at low concentrations, e.g. at 0.1%m on thiol or less, the reaction may take more time than practically acceptable, whereas the use of relatively high concentrations, e.g. above 2.0%m on thiol, naturally increases the cost of the catalyst required to produce a certain quantity of product. Hence, the preferred amount of phase transfer catalyst ranges from 0.2 to 1.0 %m on thiol.

Many thiols can be converted to the corresponding disulphide by means of the present process. Suitable thiols include aliphatic, cycloaliphatic or aromatic thiols having from 1 to 30 carbon atoms. It is evident that the thiols may contain substituents which do not interfere with the oxidation reaction, such as halogen, other aromatic or aliphatic hydrocarbyl groups, alkoxy groups or aryloxy groups. Preferably, the thiol is a hydrocarbyl thiol, in particular $C_{9-20}$-alkyl thiol and most preferred $C_{12-16}$-alkylthiol. Quite surprisingly it has been found that tertiary thiols can be oxidized in the present process at a reasonable rate, whereas in the prior art process according to J. Appl. Chem, 18 (1968), p. 239 the oxidation of t-butanethiol is extremely slow, allegedly due to steric factors. Another surprising feature of the present invention relates to the very good yield of disulphides obtained, whilst it could be expected that disulphides with an increasing size of the associated alkyl groups would be subject of hydrolysis (cf. J. Appl. Chem, 18 (1968) p. 333).

The reaction conditions prevailing in the present process can vary, and are primarily determined by economic factors. The thiol concentration is from 20 to 40%m, based on the organic solvent. The reaction temperature is preferably from 0 to 100°C, in particular from 20 to 50°C. Suitably, the reaction mixture is thoroughly mixed during the reaction so that the two liquid phases and the gas phase (air or oxygen, if used) are well dispersed.

After completion of the reaction the two liquid phases are separated. The aqueous phase may be recycled to the reaction vessel and used again as the required alkaline aqueous solution, optionally after addition of a supplementary amount of metal catalyst and/or phase transfer catalyst. The organic layer can be washed and dried, and the disulphide obtained by removal of the organic solvent.

Disulphides can be used as anti-oxidant in various polymers. Another use for disulphides, especially disulphides with alkyl groups having nine or more carbon atoms, is as additive in lubricating oils to counteract wear and oxidation. For this application the disulphide-containing organic solvent layer can be mixed directly with a lubricating oil, and the solvent subsequently removed, e.g. by subatmospheric distillation, to yield a lubricating oil (concentrate) containing the disulphide.

The invention will further be illustrated by means of Examples 2 to 8. Example 1 is a comparison example.

## EXAMPLE 1

An experiment was carried out in a mixed ether/aqueous-sodium-hydroxide-solution. 438g of n-$C_{12}$alkyl thiol (RSH) was dissolved in 2000ml ether. The solution obtained was mixed with 612.5ml of 15% sodium hydroxide solution, yielding a RSH/NaOH molar ratio of 1/1.06 . To this mixture was added 236g of iodine. The reaction was carried out under stirring at room temperature. After 4 hours a 95% conversion was determined by gas-liquid chromatography. The two liquid phases were separated, the ether layer washed with an HCl-solution and water, solids were filtered off, the solution was dried and the ether evaporated, yielding 709g of disulphide product which was of a 95% purity.

## EXAMPLES 2-7

All experiments in the following Examples 2-7 were carried out according to the following procedure. The reactions were monitored by periodically taking samples which are acidified and subjected to phase separation after which the organic layer was analysed by gas-liquid chromatography.

The thiol to be oxidized (20 g) was dissolved in 100ml of xylene. To this mixture an appropriate amount of a 15% aqueous solution of sodium hydroxide (16g NaOH in 100 ml water) was added, followed by an aqueous solution of copper sulfate (200mg $CuSO_4.5H_2O$ in 50ml water). A phase transfer catalyst was added with stirring and subsequently the oxidizing agent (air or pure oxygen) was bubbled through the reaction mixture. After completion of the reaction, the two liquid phases were separated, the organic phase was washed with an aqueous solution of hydrochloric acid and with water, any solids which were present were filtered off, the organic phase was dried and the organic solvent was removed, yielding the disulphide product.

## EXAMPLE 2

Four experiments were carried out to show the effect of the use of a phase transfer catalyst (PTC) on the oxidation rate of n-$C_{12}$-alkylthiol (RSH). The reaction was carried out in the presence of copper sulphate (1.0%m $CuSO_4.5H_2O$ based on RSH) at ambient temperature (20-22°C). The PTC used was triethylbenzylammoniumchloride (TEBAC). The product obtained was pure disulphide. The relative amounts of the reagents and the conversions are indicated in Table I.

## TABLE I

| Experiment No. | RSH/NaOH mol/mol | TEBAC %m on RSH | Reaction time, h | Conversion % |
|---|---|---|---|---|
| 2 | 1/4 | 0 | 1½ | 9 |
| | | | 4 | 18 |
| 3 | 1/4 | 0.16 | 1 | 54 |
| | | | 5 | 79 |
| | | | 20 | 100 |
| 4 | 1/4 | 0.5 | <1 | 100 |
| 5 | 1/4 | 2.5 | <1½ | 100 |

EXAMPLE 3

To show the temperature influence two experiments were carried out at different temperatures. The oxidation was established by air using 0.5%m of TEBAC, 1.0%m of $CuSO_4.5H_2O$ and a RSH/NaOH molar ratio of 1:4. The thiol used was again n-$C_{12}$-alkylthiol. The results are indicated in Table II.

## TABLE II

| Experiment No. | Temperature °C | Reaction time h. | Conversion % |
|---|---|---|---|
| 6 | ambient (20-22) | 1½ | 28 |
| 7 | 50 | 1½ | 33 |

EXAMPLE 4

To show the influence of air oxidation versus oxygen oxidation n-$C_{12}$-alkylthiol was oxidized at room temperature at a RSH/NaOH molar ratio of 1:4, in the presence of TEBAC (0.5%m) and $CuSO_4.5H_2O$ (1.0%m).

## TABLE III

| Experiment No. | Oxidizing gas | Reaction time h. | Conversion % |
|---|---|---|---|
| 8 | oxygen | <1 | 100 |
| 9 | air | 1 | 20 |
|  |  | 8 | 95 |

EXAMPLE 5

The influence of the molar ratio RSH/NaOH is shown by the results of experiments Nos. 10 and 11, in which n-$C_{12}$-alkylthiol was oxidized by oxygen at room temperature in the presence of TEBAC (0.5%m) and $CuSO_4.5H_2O$ (1.0%m).

## TABLE IV

| Experiment No. | RSH/NaOH mol/mol | Reaction time h. | Conversion % |
|---|---|---|---|
| 10 | 1/4 | <1 | 100 |
| 11 | 1/2 | 1 | 13 |
|  |  | 2 | 16 |
|  |  | 5 | 31 |

EXAMPLE 6

The effect of specific phase transfer catalysts on the oxidation has been investigated. Air was used as oxidizing gas, $n-C_{12}$-alkylthiol was the thiol to be oxidized, the amount of PTC used is indicated in Table V, and $CuSO_4.5H_2O$ was present in an amount of 1.0%m. The reaction temperature was 20-22°C. Relative amounts of RSH and NaOH are indicated in Table V and use was made of the following PTC's: TEBAC, TBA-OH (tetrabutylammoniumhydroxide), CETAB (cetyltrimethylammoniumbromide), BTMAC (benzyltrimethylammonium chloride), TBAB (tetrabutylammonium bromide), BTMA-OH (benzyltrimethylammonium hydroxide).

## TABLE V

| Experiment No. | PTC (%m) | PSH/NaOH mol/mol | Reaction time h. | Conversion % |
|---|---|---|---|---|
| 12 | TEBAC (0.5) | 1/4 | 1½ | 28 |
|  |  |  | 4½ | 48 |
|  |  |  | 6½ | 59 |
| 13 | CETAB (0.5) | 1/4 | 1½ | 31 |
|  |  |  | 4½ | 83 |
|  |  |  | 6½ | 98 |
| 14 | BTMAC (0.5) | 1/4 | 1½ | 14 |
|  |  |  | 4½ | 24 |
|  |  |  | 6½ | 33 |
| 15 | TBAB (0.5) | 1/4 | ½ | 94 |
|  |  |  | 1 | 100 |
| 16 | TBA-OH (0.5) | 1/4 | ½ | 94 |
|  |  |  | 1 | 100 |
| 17 | TBA-OH (0.2) | 1/4 | ½ | 88 |
|  |  |  | 1 | 100 |
| 18 | TBA-OH (0.5) | 1/2 | ½ | 43 |
|  |  |  | 1½ | 93 |

## TABLE V (continued)

| Experiment No. | PTC (%m) | RSH/NaOH mol/mol | Reaction time h. | Conversion % |
|---|---|---|---|---|
| 19 | TBA-OH (0.5) | 1/1 | ½ | 21 |
|  |  |  | 1½ | 37 |
|  |  |  | 3½ | 49 |
| 20 | BTMA-OH (0.5) | 1/4 | 1½ | 12 |
|  |  |  | 4½ | 19 |
|  |  |  | 6½ | 25 |

EXAMPLE 7

This example describes experiments in which thiols, different from n-$C_{12}$ alkylthiols, were subjected to an oxidation according to the invention. The experiments were carried out at room temperature in the presence of 1.0% m of $CuSO_4.5H_2O$ and at an RSH/NaOH molar ratio of 1/4. Reaction conditions and results are indicated in Table VI.

## TABLE VI

| Experiment | Oxidizing gas | PTC (%m) | RSH | Reaction time, h. | Conversion % |
|---|---|---|---|---|---|
| 21 | oxygen | TEBAC (5.0*) | $t\text{-}C_{12}H_{25}SH$ | 4 | 9 |
| | | | | 24 | 46 |
| | | | | 72 | 80 |
| 22 | air | TBA-OH (0.2) | $n\text{-}C_{16}H_{33}SH$ | 1½ | 51 |
| | | | | 4½ | 74 |

```
* 5.0%m was added as follows:
      0.5%m  initially
      2.0%m  after 4h.
      1.25%m after 24 h.
      1.25%m after 48 h.
```

EXAMPLE 8

The experimental procedure of Examples 2-7 was followed, except that the air/oxygen gaseous oxidising agent was replaced by iodine (10.9g). The thiol was $n\text{-}C_{12}SH$, (20g) in a reaction medium of xylene (100ml) and aqueous sodium hydroxide (16g NaOH in 100ml water). To this mixture copper and PTC catalysts were added (200 mg $CuSO_4 . 5H_2O$, and 250mg of 40% TBA - OH). The progress of this reaction was determined, with the following results:-

| Reaction Time (hr.) | Conversion (%) |
| --- | --- |
| ½ | 72 |
| 1 | 81 |
| 1½ | 87 |
| 2 | 93 |
| 3 | 97 |

## Claims

1. Process for the preparation of disulphides, which comprises oxidizing a thiol in an alkaline aqueous solution in the presence of a substantially water-immiscible organic solvent, a metal catalyst, and a phase transfer catalyst, the thiol concentration ranging from 20 to 40%m, based on organic solvent; after completion of reaction, separating aqueous and organic liquid phases, and isolating disulphide by removal of organic solvent from the separated organic liquid phase.

2. Process according to claim 1, in which the metal catalyst comprises cobalt, nickel or copper.

3. Process according to claim 1 or 2, in which the thiol is oxidized by means of oxygen or iodine.

4. Process according to any one of claims 1-3, in which the reaction mixture is made alkaline by means of an alkali or alkaline earth metal hydroxide or ammonia.

5. Process according to any one of claims 1-4, in which the substantially water-immiscible organic solvent is selected from (cyclo)aliphatic hydrocarbons, halogenated (cyclo)aliphatic hydrocarbons, aromatic hydrocarbons, halogenated aromatic hydrocarbons, ethers, petroleum ethers and mixtures thereof.

6. Process according to claim 5 in which the substantially water-immiscible organic solvent is benzene, toluene and/or xylene and the volume ratio of water to substantially water-immiscible organic solvent ranges from 4:1 to 1:1.

7. Process according to any one of claims 1 to 6 in which the phase transfer catalyst is selected from quaternary ammonium compounds, quaternary phosphonium compounds, crown ethers and cryptates.

8. Process according to any one of Claims 1 to 7 in which the phase transfer catalyst is selected from methyl tri($C_{8-10}$) alkylammonium halide and hydroxide, benzyltri($C_{1-2}$) ammonium halide and hydroxide, tetra($C_{1-4}$)ammonium halide and hydroxide and cetyltrimethylammonium halide and hydroxide.

9. Process according to any one of claims 1 to 8, in which the amount of phase transfer catalyst ranges from 0.1 to 2.0%m on thiol.

10. Process according to any one of claims 1-9, in which the thiol is an aliphatic, cycloaliphatic or aromatic

11

EP 0 288 104 B1

thiol having from 1 to 30 carbon atoms.

**Revendications**

1. Procédé pour la préparation de disulfures, qui comprend l'oxydation d'un thiol dans une solution aqueuse alcaline en présence d'un solvant organique substantiellement non-miscible avec l'eau, d'un catalyseur métallique et d'un catalyseur à transfert de phase, la concentration du thiol étant comprise entre 20 et 40 moles % par rapport au solvant organique, et, après la fin de la réaction, la séparation des phases liquides aqueuse et organique et l'isolement du disulfure par élimination du solvant organique de la phase liquide organique séparée.

2. Procédé selon la revendication 1, dans lequel le catalyseur métallique comprend du cobalt, du nickel ou du cuivre.

3. Procéeé selon la revendication 1 ou 2, dans lequel le thiol est oxydé au moyen d'oxygène ou d'iode.

4. Procédé selon l'une quelconque des revendications 1-3, dans lequel le mélange réactionnel est rendu alcalin au moyen d'un hydroxyde de métal alcalin ou alcalinoterreux ou d'ammoniaque.

5. Procédé selon l'une quelconque des revendications 1-4, dans lequel le solvant organique substantiellement non-miscible avec l'eau est choisi parmi des hydrocarbures (cyclo)aliphatiques, des hydrocarhures (cyclo)aliphatiques halogénés, des hydrocarbures aromatiques, des hydrocarbures aromatiques halogénés, des éthers, des éthers de pétrole et leurs mélanges.

6. Procédé selon la revendication 5, dans lequel le solvant organique substantiellement non-miscible avec l'eau est du benzène, du toluène et/ou du xylène et le rapport en volume de l'eau au solvant organique substantiellement non-miscible avec l'eau est compris entre 4:1 et 1:1.

7. Procédé selon l'une quelconque des revendications 1 à 6, dans lequel le catalyseur à transfert de phase est choisi parmi des composés d'ammonium quaternaire, des composés de phosphonium quaternaire, des étherscouronne et des cryptates.

8. Procédé selon l'une quelconque des revendications 1 à 7, dans lequel le catalyseur à transfert de phase est choisi parmi les halogénures et hydroxydes de tri($C_{8-10}$) alcoylammonium, les halogénures et hydroxydes de benzyltri($C_{1-2}$) alcoylammonium, les halogénures et hydroxydes de tétra ($C_{1-4}$) alcoylammonium et les halogénures et l'hydroxyde de cétyltriméthylammonium.

9. Procédé selon l'une quelconque des revendications 1 à 8, dans lequel la quantité de catalyseur à transfert de phase est comprise entre 0,1 et 2,0 moles % par rapport au thiol.

10. Procédé selon l'une quelconque des revendications 1 à 9, dans lequel le thiol est un thiol aliphatique, cycloaliphatique ou aromatique ayant de 1 à 30 atomes de carbone.

**Patentansprüche**

1. Verfahren zur Herstellung von Disulfiden, welches ein Oxidieren eines Thiols in einer alkalischen wäßrigen Lösung in Gegenwart eines im wesentlichen wasserunmischbaren organischen Lösungsmittels, eines Metallkatalysators und eines Phasentransferkatalysators, wobei die Thiolkonzentration von 20 bis 40 Masse-%, bezogen auf organisches Lösungsmittels, beträgt, nach beendeter Umsetzung ein Trennen der wäßrigen und der organischen flüssigen Phasen und ein Isolieren von Disulfid durch Abtrennen des organischen Lösungsmittels von der abgetrennten organischen flüssigen Phase umfaßt.

2. Verfahren nach Anspruch 1, worin der Metallkatalysator Kobalt, Nickel oder Kupfer enthält.

3. Verfahren nach Anspruch 1 oder 2, worin das Thiol mittels Sauerstoff oder Iod oxidiert wird.

4. Verfahren nach den Ansprüchen 1 bis 3, worin das Reaktionsgemisch durch ein Alkali- oder Erdalkalimetallhydroxid oder durch Ammoniak alkalisch gemacht wird.

12

5. Verfahren nach einem der Ansprüche 1 bis 4, worin das im wesentlichen wasserunmischbare organische Lösungsmittel unter (cyclo)aliphatischen Kohlenwasserstoffen, halogenierten (cyclo)aliphatischen Kohlenwasserstoffen, aromatischen Kohlenwasserstoffen, halogenierten aromatischen Kohlenwasserstoffen, Ethern, Petrolethern und Gemischen hievon ausgewählt wird.

6. Verfahren nach Anspruch 5, worin das im wesentlichen wasserunmischbare organische Lösungsmittel Benzol, Toluol und/oder Xylol ist und das Volumenverhältnis von Wasser zu dem im wesentlichen wasserunmischbaren organischen Lösungsmittel von 4:1 bis 1:1 beträgt.

7. Verfahren nach einem der Ansprüche 1 bis 6, worin der Phasentransferkatalysator unter quaternären Ammoniumverbindungen, quaternären Phosphoniumverbindungen, Kronenethern und Cryptaten ausgewählt wird.

8. Verfahren nach einem der Ansprüche 1 bis 7, worin der Phasentransferkatalysator unter Methyl-tri-($C_{8-10}$)alkylammoniumhalogenid und -hydroxid, Benzyltri($C_{1-2}$)alkylammoniumhalogenid und -hydroxid, Tetra($C_{1-4}$)alkylammoniumhalogenid und -hydroxid und Cetyltrimethylammoniumhalogenid und -hydroxid ausgewählt wird.

9. Verfahren nach einem der Ansprüche 1 bis 8, worin die Menge an Phasentransferkatalysator im Berich von 0,1 bis 2,0 Masse-%, bezogen auf Thiol, liegt.

10. Verfahren nach einem der Ansprüche 1 bis 9, worin das Thiol ein aliphatisches, cycloaliphatisches oder aromatisches Thiol mit 1 bis 30 Kohlenstoffatomen ist.